# FASCICULE DE BREVET EUROPEEN

(11) **EP 3 841 159 B1**
(45) Date de publication et mention de la délivrance du brevet: **06.12.2023**
(21) Numéro de dépôt: 19758942.7
(22) Date de dépôt: 21.08.2019
(51) Int. Cl.: A61L 27/34, A61L 29/10, A61L 31/08, B05D 5/08, C08J 7/06, C09D 105/08, F16N 1/00, C08J 7/04, C08L 5/08, C08B 37/08

(54) **PROCÉDÉ DE LUBRIFICATION**
SCHMIERVERFAHREN
LUBRICATION METHOD

(30) Priorité: 22.08.2018 FR 1857583
(43) Date de publication de la demande: 30.06.2021
(73) Titulaire: NATVI, 92200 Corbeil-Essonnes (FR)
(72) Inventeur: DAO, Vi Thuy, 94260 FRESNES (FR)
(74) Mandataire: LLR
(86) Numéro de dépôt international: PCT/EP2019/072402
(87) Numéro de publication internationale: WO 2020/039008

(56) Documents cités:
- EP-A2- 0 161 887
- FR-A1- 3 001 642
- US-A1- 2009 253 599
- S. KHUNMANEE ET AL.: "Crosslinking method of hyaluronic-based hydrogel for biomedical applications", JOURNAL OF TISSUE ENGINEERING, vol. 8, 2017, pages 1-16, XP002791499,

## Description

L'invention concerne un procédé de lubrification.

La fonctionnalisation de la surface des matériaux utilisés pour diverses applications, notamment dans le domaine médical, est un facteur déterminant pour l'interaction des matériaux avec l'environnement. Toutefois, une telle modification n'est pas toujours simple.

Les dispositifs médicaux peuvent être recouverts avec une multitude de compositions ou composés selon les utilisations envisagées. Il peut notamment être nécessaire de recouvrir un dispositif médical avec une composition comprenant un agent pharmaceutique ou un agent permettant l'hydratation, notamment dans le cadre d'une implantation dans un organisme. De la même manière, l'utilisation d'un dispositif médical peut être améliorée en le recouvrant d'une composition lubrifiante.

Il est toutefois souvent nécessaire que ces types de compositions soient à la fois biocompatible et possèdent un bon maintien sur la surface sur laquelle ces compositions sont déposées.

On connaît déjà de l'art antérieur la demande de brevet FR 3001642 qui décrit un procédé de recouvrement d'une surface hydrophobe avec un composé hydrophile. Le procédé décrit dans ce document implique une superposition d'une première couche d'un composé amphiphile présentant des propriétés d'auto-assemblage et d'une seconde couche d'un polymère hydrophile, en particulier des polysaccharides.

Un tel procédé s'est avéré très utile pour rendre hydrophile une surface hydrophobe. Toutefois, ce procédé ne permet pas de fournir des surfaces qui seraient à la fois biocompatibles et qui seraient utiles pour la lubrification suffisamment durable des surfaces, notamment pour favoriser le glissement répétitif d'une autre surface sur cette surface recouverte. Le document US2009/253599 décrit la réticulation de polysaccharides au moyen de différents agents de réticulation, et notamment un chélate de titane. Toutefois, l'objectif de ce document ne concerne en rien la lubrification. Le document de S. Khunmanee et al. 2017, J. of Tissue Engineering, vol. 8, pages 1-16 ; divulgue une large gamme d'agents de réticulation pour réticuler l'acide hyaluronique dans un but d'utilisation médicale. La demande européenne EP 0161887 décrit de l'acide hyaluronique réticulé avec un composé epoxy polyfonctionnel choisi parmi les composés halogènomethyloxiranes, et un éther diglycidique de bisphénol A ou de bisphénol.

Aussi, existe-t-il un besoin d'améliorer le procédé de l'art antérieur, en particulier du fait de la relative fragilité du revêtement résultant et de l'absence de résistance au frottement de celui-ci, malgré sa grande biocompatibilité.

Un des moyens de stabiliser les structures consiste en la réticulation de la couche de polymères hydrophiles.

Toutefois, les agents de réticulation couramment utilisés sont soit instables dans les solutions aqueuses, soit conduisent à la formation de polymères réticulés très peu lubrifiants .

L'invention a donc pour objectif de pallier ces inconvénients.

L'un des buts de l'invention est de fournir un procédé de lubrification suffisamment durable d'une structure, notamment constituée de polymères réticulés.

Un autre but de l'invention est de fournir un élément lubrifié obtenu par ledit procédé.

Encore un autre but de l'invention est de fournir une méthode de lubrification d'une surface qui ne l'est structurellement pas de manière simple et rapide, ainsi que la surface qui en résulte. L'invention est limitée à la portée des revendications 1 à 10.

Il est décrit, en dehors du cadre de l'invention, une méthode de lubrification d'un élément essentiellement constitué d'un polymère hydrophile réticulé, ladite méthode comprenant :
a) une étape de mise en contact dudit élément essentiellement constitué d'un polymère hydrophile réticulé avec une solution d'un polymère hydrophile libre, ou non réticulé, ledit élément essentiellement constitué d'un polymère hydrophile réticulé ayant été réticulé au moyen d'un agent de réticulation qui est un chélate de métal de transition soluble et stable dans l'eau.

La réticulation de polymères hydrophiles par un chélate de métal de transition soluble et stable dans l'eau permet non seulement d'obtenir une structure polymérique dudit polymère hydrophile rigidifié, notamment sous forme de gel, mais également que la réticulation de la structure polymérique par un chélate de métal de transition soluble dans l'eau permet de maintenir une couche de polymères hydrophile non réticulé qui confère ainsi des propriétés lubrifiantes à ladite structure polymère hydrophile rigidifiée.

Les agents de réticulation peuvent inclure n'importe quel composé d'un métal de transition tel que titane, zirconium, chrome ou hafnium. Les agents de réticulation les plus appropriés sont des ions complexes issus des tetramethyl orthotitanate, tetraethyl titanates, tetrapropyl titanates, tetrapropyl zirconates, tetrabutyl zirconates qui sont rendus solubles dans l'eau par la réaction avec des ligands tels que des composés béta-diceto, des amino alcools, des hydroxylamines, des alcools ethoxylés, des lactones et des acides polyacryliques. Sont cités des exemples plus spécifiques pour les réactions avec des ligands tels que triethanolamine, l'acetylacetonate et l'acide lactique...

Le polymère hydrophile selon l'invention peut être, sans que cela soit limitatif, formé de polymères anioniques ou cationiques, des polymères acides, des polymères d'amines ou d'acides aminés, et des sels biologiquement et/ou pharmaceutiquement acceptables de ceux-ci. Il est possible également de disposer de mélanges des polymères susmentionnés. On peut citer comme exemples de polymères utilisables dans l'invention : le collagène, le collagène modifié par oxydation, les polysaccharides, les alginates, l'acide hyaluronique, la polylysine ... De tels exemples sont à titre indicatif et ne doivent pas être considérés comme limitant la portée de l'invention.

Le polymère hydrophile peut être choisi parmi des polycations, par exemple des polyamines, des histones (protéines entourant l'ADN des cellules eucaryotes et riches en acides aminés basiques), du chitosan, de la polylysine...

Les mucopolysaccharides ou les glycoprotéines sont également des polymères hydrophiles utilisables.

Il est également décrit une méthode de lubrification d'un élément essentiellement constitué d'un polymère hydrophile réticulé, ladite méthode comprenant :
a) une étape de mise en contact dudit élément essentiellement constitué d'un polymère hydrophile réticulé avec une solution d'acide hyaluronique libre, ou non réticulé, ledit élément essentiellement constitué d'un polymère hydrophile réticulé ayant été réticulé au moyen d'un agent de réticulation qui est un chélate de métal de transition soluble et stable dans l'eau.

Il est par ailleurs décrit une méthode de lubrification d'un élément essentiellement constitué d'acide hyaluronique réticulé, ladite méthode comprenant :
b) une étape de mise en contact dudit élément essentiellement constitué d'acide hyaluronique réticulé avec une solution d'un polymère hydrophile libre, ou non réticulé, ledit élément essentiellement constitué d'acide hyaluronique réticulé ayant été réticulé au moyen d'un agent de réticulation qui est un chélate de métal de transition soluble et stable dans l'eau.

L'invention concerne une méthode de lubrification d'un élément essentiellement constitué d'acide hyaluronique réticulé, ladite méthode comprenant :
a) une étape de mise en contact dudit élément essentiellement constitué d'acide hyaluronique réticulé avec une solution d'acide hyaluronique libre, ou non réticulé, ledit élément essentiellement constitué d'acide hyaluronique réticulé ayant été réticulé au moyen d'un agent de réticulation qui est un chélate de titane ou de zirconium soluble et stable dans l'eau.

L'invention repose dans l'un de ses aspects sur la constatation surprenante faite par les inventeurs que la réticulation de l'acide hyaluronique par un chélate de titane soluble et stable dans l'eau permet non seulement d'obtenir une structure polymérique d'acide hyaluronique rigidifiée, notamment sous forme de gel, mais également que la réticulation de la structure polymérique par un chélate de titane soluble dans l'eau permet de maintenir une couche d'acide hyaluronique non réticulé qui confère ainsi des propriétés lubrifiantes à ladite structure d'acide hyaluronique rigidifiée.

Plus particulièrement, les inventeurs ont constaté de manière surprenante que les chélates de Ti solubles dans l'eau étaient de bons agents de réticulation de l'acide hyaluronique et de bons agents permettant la rétention d'acide hyaluronique libre sur une structure réticulée avec ledit chélates de Ti soluble dans l'eau.

Il est à noter que les chélates de Ti insolubles dans l'eau ou qui se dégradent une fois solubilisés dans l'eau (c'est-à-dire qui ne sont pas stables dans l'eau) ne permettent pas de mettre en oeuvre l'invention, et sont donc de fait exclus de la présente invention.

L'une des problématiques des agents de réticulation est leur solubilité dans l'eau. En effet, beaucoup d'agents de réticulation sont connus pour être solubles dans les solvants organiques mais être très instables dans l'eau. Or, il est essentiel que l'agent de réticulation utilisé dans l'invention soit soluble, et actif dans l'eau, sans quoi il n'est pas possible de les utiliser pour réticuler l'acide hyaluronique qui lui, est hydrosoluble.

Selon le procédé susmentionné, on utilise simplement un élément essentiellement constitué d'acide hyaluronique réticulé par ledit agent de réticulation, qui a préférablement été rincé à l'eau au préalable, et on y dépose une solution d'acide hyaluronique. La réticulation par du chélate de titane permet à l'acide hyaluronique non réticulé d'être retenu à la surface de la structure réticulée.

A l'issue de ce procédé très simple et respectueux de l'environnement, est obtenue une structure composée de deux couches : une couche d'acide hyaluronique réticulé recouverte par une couche d'acide hyaluronique non réticulé.

Il est notamment avantageux que l'acide hyaluronique ajouté à l'étape a) soit de l'acide hyaluronique à une concentration de 0,1 à 0,5% dans l'eau (masse/volume ou m/v), notamment de 0,2 à 0.3% (m/v) dans l'eau.

Dans un mode de réalisation avantageux, ledit agent de réticulation soluble et stable dans l'eau est un chélate de formule 1 suivante : 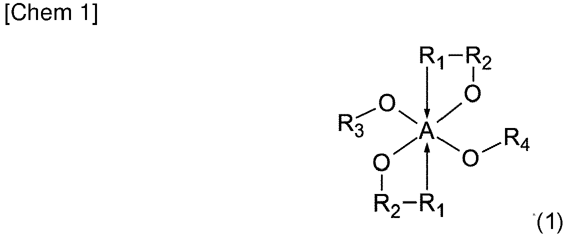 où A est Ti, et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique, comprenant un groupe méthyle, un groupe éthyle, un groupe propyle ou méthyléthyle ou cyclopentyle, un groupe butyle ou isopropyle ou sec-butyle ou tert-butyle ou cyclobutyle.

Les composés ou agents réticulants particulièrement avantageux selon l'invention répondent donc à la formule 1a suivante : 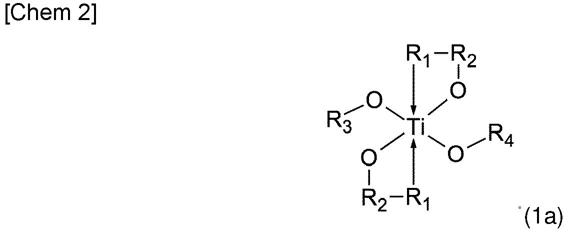 où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique, comprenant un groupe méthyle, un groupe éthyle, un groupe propyle ou méthyléthyle ou cyclopentyle, un groupe butyle ou isopropyle ou sec-butyle ou *tert*-butyle ou cyclobutyle.

De manière encore plus avantageuse, ledit agent de réticulation est l'un des composés suivants : 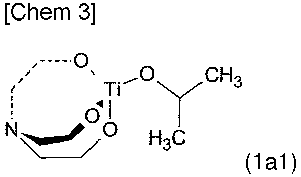 ou Titanium(IV) (triethanolaminato)isopropoxide (La triéthanolamine complexe de titane Tyzor TE) no CAS : 74665-17-1, 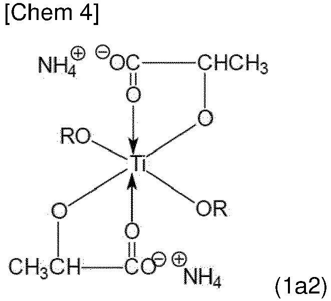 D'ihydroxybis(ammonium lactato)titanium(IV) - TYZOR^{®} LA-lactic acid titanate chelate, ammonium sait no CAS 65104-06-5, et

Dans un mode de réalisation avantageux, l'invention concerne la méthode susmentionnée, où ledit agent de réticulation est l'agent de réticulation de formule 1a1 suivante :

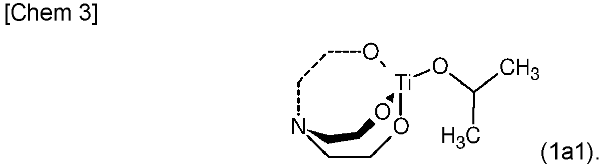

Le composé de formule 1a1 est également connu sous le nom de Tyzor TE, notamment commercialisé par la société DuPont ou triéthanolamine complexe de titane (no CAS 74665-17-1).

Dans un autre mode de réalisation avantageux, l'invention concerne la méthode susmentionnée, où le dit agent de réticulation est ou l'agent de réticulation de formule 1b suivante :

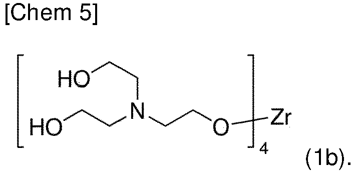

Il est également décrit ici, en dehors du cadre de l'invention, que la méthode susmentionnée, peut comprendre, en outre :
b) une étape de réticulation du polymère hydrophile libre, ou non réticulé, mis en contact avec ladite composition essentiellement constituée de polymère hydrophile réticulé à l'aide d'un agent de réticulation susmentionné, notamment de formule 1, pour obtenir une seconde couche de composition essentiellement constituée de polymère hydrophile réticulé, et
c) une étape de mise en contact de la seconde couche de composition essentiellement constituée de polymère hydrophile réticulé avec une solution de polymère hydrophile libre.

De même la méthode susmentionnée peut comprendre, en outre :
b) une étape de réticulation de l'acide hyaluronique libre, ou non réticulé, mis en contact avec ladite composition essentiellement constituée de polymère hydrophile réticulé à l'aide d'un agent de réticulation susmentionné, notamment de formule 1a, pour obtenir une seconde couche de composition essentiellement constituée d'acide hyaluronique réticulé, et
c) une étape de mise en contact de la seconde couche de composition essentiellement constituée d'acide hyaluronique réticulé avec une solution
   - soit d'acide hyaluronique libre,
   - soit de polymère hydrophile libre.

On peut également décrire ici la méthode susmentionnée qui peut comprendre, en outre :
b) une étape de réticulation du polymère hydrophile libre, ou non réticulé, mis en contact avec ladite composition essentiellement constituée de l'acide hyaluronique réticulé à l'aide d'un agent de réticulation susmentionné, notamment de formule 1a, pour obtenir une seconde couche de composition essentiellement constituée de polymère hydrophile réticulé, et
c) une étape de mise en contact de la seconde couche de composition essentiellement constituée de polymère hydrophile réticulé avec une solution
   - soit d'acide hyaluronique libre,
   - soit de polymère hydrophile libre.

Dans ce qui précède, ledit polymère hydrophile est avantageusement un bio polymère hydrophile lubrifiant tel que mucopolysaccharide ou glycoprotéine lubrifiante.

Dans un mode de réalisation avantageux, l'invention concerne la méthode susmentionnée, comprenant, en outre :
b) une étape de réticulation de l'acide hyaluronique libre, ou non réticulé, mis en contact avec ladite composition essentiellement constituée d'acide hyaluronique réticulé à l'aide d'un agent de réticulation de formule 1 pour obtenir une seconde couche de composition essentiellement constituée d'acide hyaluronique réticulé, et
c) une étape de mise en contact de la seconde couche de composition essentiellement constituée d'acide hyaluronique réticulé avec une solution d'acide hyaluronique libre.

Afin de stabiliser la structure obtenue après l'étape a) du procédé selon l'invention, il peut être avantageux de réticuler, en utilisant le composé réticulant susmentionné, la couche d'acide hyaluronique libre qui a été déposée sur l'élément essentiellement constitué d'acide hyaluronique réticulé telle que définie ci-dessus. Afin de lubrifier cette seconde couche de lubrification, il est alors déposé sur la dernière couche d'acide hyaluronique réticulé à l'étape b) une couche d'acide hyaluronique libre ou non réticulé.

La structure ainsi obtenue est constituée de deux couches d'acide hyaluronique réticulé directement en contact mais distinctes, et une couche d'acide hyaluronique non réticulé.

De manière avantageuse, les étapes b) et c) sont répétées au moins une fois.

La répétition des étapes b) et c) a pour objectif de stabiliser les structures ainsi obtenues, tout en préservant, avec l'étape c) un élément lubrifié du fait de la présence de la couche d'acide hyaluronique libre.

Dans le cas d'une répétition des étapes b) et c), la structure aura 3 couches d'acide hyaluronique réticulé, la dernière couche étant recouverte par une couche d'acide hyaluronique libre ou non réticulé ou d'une couche d'un bio polymère hydrophile lubrifiant, non réticulé.

Il est décrit ci-après, en dehors du cadre de l'invention, un élément lubrifié essentiellement constitué d'au moins une couche d'un polymère hydrophile, notamment un polymère hydrophile susmentionné, réticulé à l'aide d'un agent de réticulation tel que défini ci-dessus, ladite au moins une couche d'un polymère hydrophile étant recouverte d'une une couche de polymère hydrophile non réticulé, notamment d'une couche d'un bio polymère hydrophile lubrifiant non réticulé.

Un élément lubrifié essentiellement constitué d'au moins une couche d'un polymère hydrophile est également décrit, notamment un polymère hydrophile susmentionné, réticulé à l'aide d'un agent de réticulation tel que défini ci-dessus, ladite au moins couche d'un polymère hydrophile étant recouverte d'une une couche d'acide hyaluronique non réticulé.

L'invention concerne également un élément lubrifié essentiellement constitué d'au moins une couche d'acide hyaluronique réticulé à l'aide d'un agent de réticulation qui est un chélate de titane soluble et stable dans l'eau, ladite au moins une couche d'acide hyaluronique réticulé étant recouverte d'une couche d'acide hyaluronique non réticulé, ledit élément lubrifié étant susceptible d'être obtenu selon le procédé défini ci-dessus dans l'invention.

Cet élément est à la fois relativement solide grâce à la réticulation, et souvent sous la forme d'un gel, et présente une bonne lubrification du fait de la présence sur sa couche supérieure externe d'acide hyaluronique libre ou non réticulé notamment d'une couche d'un bio polymère hydrophile lubrifiant non réticulé.

.

On peut décrire ici un procédé de lubrification d'une surface hydrophobe, ou d'un support hydrophobe, comprenant :
a. une étape de mise en contact de la surface hydrophobe en vue de son recouvrement avec une première composition comprenant
   i. un solvant, et
   ii. un soluté consistant en un composé amphiphile capable de s'auto assembler et d'interagir avec ladite surface, l'auto-assemblage dudit composé et l'interaction avec la surface se faisant au moyen de liaisons différentes de liaisons covalentes ou ioniques, ledit solvant étant compatible avec ledit composé et ladite surface hydrophobe,
b. une étape subséquente de rinçage avec une solution aqueuse de ladite surface hydrophobe recouverte à l'étape précédente,
c. une étape de mise en contact de la surface rincée avec une seconde composition hydrophile comprenant un polymère hydrophile,
d. une étape de réticulation du polymère hydrophile présent sur la surface obtenue à l'étape précédente à l'aide d'un agent de réticulation pour obtenir une première couche de polymère hydrophile réticulé, et
e. une étape de mise en contact de la première couche de polymère réticulé avec une solution de polymère hydrophile libre, où ledit agent de réticulation est un chélate de métal de transition, notamment de titane ou de zirconium ou de hafnium soluble et stable dans l'eau, notamment un chélate de formule 1 suivante : 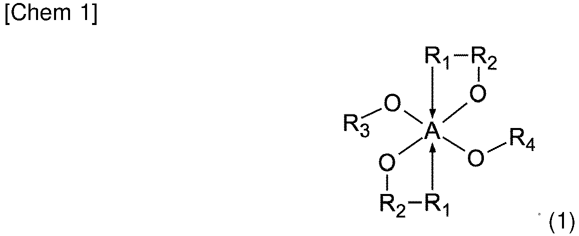 Où A est Ti où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique.

Dans l'invention, on entend par « surface hydrophobe » la surface ou une surface d'un dispositif médical. Par « support hydrophobe » on entend également une face ou une partie d'un dispositif médical.

Ainsi, dans l'invention, la « surface » ou le « support » correspond à un dispositif médical solide, en particulier qui est susceptible de frotter en pénétrant le corps humain ou animal. Par exemple sans être limitatif, le support peut être : l'intérieur d'une seringue, le piston d'une seringue, le caoutchouc du piston d'une seringue, une aiguille creuse utilisée ou non avec une seringue (qu'il s'agisse de la surface externe de l'aiguille ou du canal de celle-ci), un stent, un cathéter (notamment à usage intracérébral et intracardiaque, vasculaire ainsi que les fils de guidage vasculaires), une lentille (notamment une lentille destinée à être insérée dans l'oeil), une sonde, une mèche, et plus généralement tout instrument médical ou ancillaire chirurgical destiné à permettre la pose d'implant, une prothèse apte à être insérée dans le corps humain ou animal de manière temporaire ou définitive, une valve cardiaque, un stimulateur cardiaque notamment artificiel, un implant orthopédique ....

Dans l'invention le terme « dispositif médical » prend le sens légal défini par la loi française, à savoir tout instrument, appareil, équipement, matière, produit, manufacturé, à l'exception des produits d'origine humaine ou animale, ou autre article utilisé seul ou en association, destiné par le fabricant à être utilisé chez l'homme à des fins médicales et dont l'action principale voulue n'est pas obtenue par des moyens pharmacologiques ou immunologiques ni par métabolisme, mais dont la fonction peut être assistée par de tels moyens.

Les surfaces hydrophobes à recouvrir par le procédé de l'invention sont généralement en matériaux polymères du type thermoplastique comme le polyéthylène (PE), le polypropylène (PP), le polyéthylènetéréphtalate (PET), le polybutylène théréphtalate (PBT), le polyoxyméthylène (POM), le polyarylate (PAr), polyéthercétone (PEK), les polymères fluorés (par exemple : polyfluorure de vinylidène (PVDF), les polymères cyclo-oléfiniques (COC) (par exemple les polymères commercialisés sous la marque « TOPAS »), les copolymères cyclooléfiniques (COP) (par exemple les copolymères commercialisés sous la marque «Zeonex»), polytétrafluoroéthylène (PTFE), le polychlorotrifluoroéthylène (PCTFE)), polysulfone (PSU), l'éthylène-propylène-diène monomère (EPDM), ou de type thermodurcissable comme les caoutchoucs synthétiques (par exemple: les caoutchouc halogénobutyles, les caoutchoucs nitriles, les polyisoprènes, et les polychloroprène) ou encore du type élastomères thermoplastiques (par exemple : le copolymère EPDM-PP commercialisé sous la marque « Santoprène », le copolymère bloc styrène-éthylène-butylène-styrène (SEBS), etc.).

D'autres surfaces telles que l'acier inoxydable, l'or, le titane, le platine, l'aluminium, l'alliage de nickel et de titane ou nitinol, le tantale, les silicones peuvent être recouvertes selon le procédé selon l'invention. La liste susmentionnée n'est pas limitative, et l'homme de métier comprend qu'il s'agit des surfaces hydrophobes utilisables notamment dans le cadre des dispositifs à usage médical susmentionnés.

On peut également décrire ici, en dehors du cadre de l'invention, un procédé de lubrification d'une surface hydrophobe d'un dispositif médical susceptible de frotter en pénétrant le corps humain, ledit procédé comprenant :
a. une étape de mise en contact de la surface hydrophobe dudit dispositif médical, en vue de son recouvrement, avec une première composition comprenant
   i. un solvant, et
   ii. un soluté consistant en un composé amphiphile capable de s'auto assembler et d'interagir avec ladite surface, l'auto-assemblage dudit composé et l'interaction avec la surface dudit dispositif médical se faisant au moyen de liaisons différentes de liaisons covalentes ou ioniques,
   ledit solvant étant compatible avec ledit composé et ladite surface hydrophobe dudit dispositif médical,
b. une étape subséquente de rinçage avec une solution aqueuse de ladite surface hydrophobe dudit dispositif médical recouverte à l'étape précédente,
c. une étape de mise en contact de la surface rincée avec une seconde composition hydrophile comprenant un polymère hydrophile,
d. une étape de réticulation du polymère hydrophile présent sur la surface obtenue à l'étape précédente à l'aide d'un agent de réticulation pour obtenir une première couche de polymère hydrophile réticulé, et
e. une étape de mise en contact de la première couche de polymère réticulé avec une solution de polymère hydrophile libre,
où ledit agent de réticulation est un chélate de métal de transition, notamment de titane ou de zirconium ou de hafnium soluble et stable dans l'eau, notamment un chélate de formule 1 suivante : 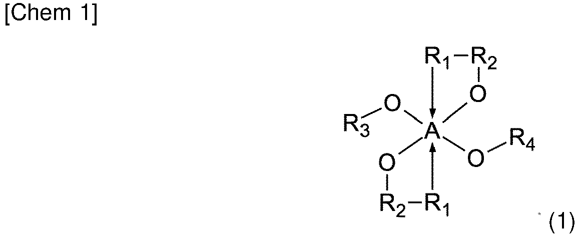 Où A est Ti où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique.

La réticulation d'un polymère hydrophile stabilise la structure avec le composé amphiphile auto-assemblable de sorte que la couche de polymère hydrophile n'est pas éliminée par simple frottement. Si l'on ajoute une couche supplémentaire de composé hydrophile, la la surface est rendue lubrifiée.

Ceci n'est possible qu'avec l'utilisation d'agents de réticulation solubles et stables, et actifs, dans une solution aqueuse. En effet, la première étape de recouvrement du support étant le dépôt de la composition amphiphile sur le support, cette dernière serait solubilisée dans un solvant organique qui servirait à dissoudre l'agent de réticulation. Aussi, un agent de réticulation de la composition hydrophile qui ne serait soluble que dans un solvant organique aurait pour conséquence de décaper la couche de composé amphiphile entrainant ainsi le polymère hydrophile, et donc de ne plus permettre de rendre hydrophile le support hydrophobe.

Le composé utilisé dans le cadre de l'étape a) de l'invention est un composé amphiphile, c'est-à-dire un composé qui possède à la fois au moins un groupe hydrophile et au moins un groupe hydrophobe.

L'auto assemblage moléculaire est le phénomène par lequel des composés forment par eux-mêmes des structures avec un haut degré d'organisation, sans intervention externe. On peut schématiquement considérer que ces composés possèdent une affinité suffisante pour qu'ils s'assemblent entre eux.

On distingue deux types d'auto-assemblage, intramoléculaire et intermoléculaire. L'auto-assemblage intramoléculaire produit souvent des polymères complexes qui ont la possibilité d'adopter une conformation de structure stable et bien définie. L'auto-assemblage intermoléculaire définit la capacité que certaines molécules ont de former des assemblages supramoléculaires. Un exemple de ce type d'auto assemblage concerne la formation de micelles à partir de surfactants en solution. L'auto assemblage est généralement dû à des liaisons de type van der Waals. La stabilité de l'auto assemblage résulte en grande partie de facteurs thermodynamiques qui privilégient les formes ordonnées de la matière (néguentropie) par rapport aux états désordonnés.

L'auto-assemblage peut se produire spontanément, par exemple dans les cellules (où la membrane est faite d'une double couche lipidique auto-assemblée) et d'autres systèmes biologiques, tout comme dans les systèmes artificiels. Il en résulte généralement une augmentation de l'organisation interne du système. Les systèmes biologiques auto-assemblés, incluant les peptides synthétiques auto-assemblés et d'autres biomatériaux, montrent une plus grande facilité de manipulation, de biocompatibilité et de fonctionnalité. Ces avantages sont dus directement à l'auto-assemblage à partir de précurseurs biocompatibles qui créent des biomatériaux synthétisés à l'échelle nanométrique.

Dans la première étape a) du procédé, seules les interactions intermoléculaires sont mises en jeu. Les forces causées par l'auto assemblage de ces composés sont suffisantes pour permettre au composé d'interagir avec le substrat, et de rester déposé uniformément sur celui-ci, sans qu'il soit nécessaire de faire intervenir des interactions physico-chimiques avec celui-ci.

En d'autres termes, il n'existe pas de liaison covalente entre le composé auto assemblable et la surface recouverte par celui-ci. De la même manière, il n'existe pas de liaisons ioniques entre le composé auto assemblable et la surface recouverte par celui-ci.

Dans la première étape du procédé selon l'invention, une solution de solvant comprenant un composé auto assemblable est mise en contact avec la surface à recouvrir. « ledit solvant est compatible avec ledit composé et ladite surface hydrophobe » ce qui signifie que le composé auto assemblable est soluble dans ledit solvant, et que le solvant n'exerce pas d'effet altérant drastiquement la nature et la surface du substrat à recouvrir.

Dans un mode de réalisation avantageux, le solvant utilisé est susceptible d'exercer une légère abrasion de la surface à recouvrir, de sorte que des micro-dépressions peuvent être provoquées à la surface de ladite surface.

Avantageusement, la surface est préalablement nettoyée et dégraissée afin d'éliminer des traces de composés pouvant altérer l'auto assemblage des composés.

Selon la nature de la surface, elle peut être nettoyée et dégraissée avec un solvant approprié. L'homme du métier est capable de déterminer le solvant ou les mélanges de solvants requis. La partie exemple, ci-après donne certaines indications.

Dans l'invention, on entend par « solvant compatible avec ledit composé» un solvant capable de solubiliser ledit composé amphiphile auto assemblable. L'homme du métier peut aisément déterminer le solvant, ou les mélanges de solvants les plus appropriés pour solubiliser le composé amphiphile. On entend par « solvant compatible avec ladite surface hydrophobe» un solvant capable de ne pas dégrader ou altérer la surface hydrophobe.

Dans un autre mode de réalisation de l'invention, la première étape du procédé peut être mise en oeuvre directement lors de la fabrication de la surface hydrophobe à recouvrir. Par exemple, le composé auto assemblable, considéré comme un additif sous la forme de poudre ou de granules, éventuellement sous forme d'une solution, notamment d'une solution fortement concentrée, est ajouté à des granulés de polymères hydrophobes, le mélange ainsi obtenu est alors soumis à une fusion puis une extrusion. Dans ce mode de réalisation, lors du refroidissement, les composés auto assemblables migrent lentement vers les parties superficielles de la surface en formation, et les parties polaires du composé sont exposées à l'extérieur de la surface.

Dans encore un autre mode de réalisation avantageux, la surface à recouvrir est traitée avec un solvant du polymère, pendant un temps déterminé, de telle sorte qu'une micro abrasion apparait sur les parties superficielles de la surface. De telles abrasions permettent la diffusion et le regroupement du composé auto assemblable et l'exposition de sa partie hydrophile vers l'extérieur du substrat. Dans la seconde étape b) du procédé, la couche de composé amphiphile est lavée avec une solution aqueuse. Une fois lavée, la surface peut avantageusement être séchée.

Dans la troisième étape c) du procédé selon l'invention, la surface hydrophobe, recouverte par le composé amphiphile auto assemblable qui a été lavée, et éventuellement séchée est recouverte, ou enduite, par une seconde composition comprenant au moins un, ou plusieurs, polymère hydrophiles.

Hors contexte de l'invention, la seconde composition comprenant au moins un, ou plusieurs, polymères hydrophiles est classiquement mise en solution, et mise en contact avec la surface préalablement lavée et éventuellement séchée à l'étape précédente.

Il est avantageux de choisir le polymère hydrophile de sorte qu'il présente une affinité pour le composé auto assemblable amphiphile.

Aussi, on peut des polycations, par exemple des polyamines, des histones (protéines entourant l'ADN des cellules eucaryotes et riches en acides aminés basiques), du chitosan, de la polylysine... lorsque le composé auto assemblable comprend une partie polaire anionique et de choisir des polyanions, comme les glycosaminoglycans, tels que le sulfate de chondroitine, l'héparine ou les sulfates d'héparan, les sulfates de kératines, le sulfate de dermatan, et l'acide hyaluronique, des polypeptides notamment synthétiques tels que les peptides poly acide glutamique, poly acide aspartique..., lorsque le composé auto assemblable comprend une partie polaire cationique.

Avantageusement, le polymère hydrophile interagit avec le composé amphiphile auto assemblable au moyen de liaisons ioniques.

L'invention concerne en outre une méthode de lubrification d'un support hydrophobe comprenant :
a) une étape de recouvrement du support avec une première composition comprenant un solvant, et
   un soluté choisi parmi la stéarylamine ou l'acide stéarique, pour l'obtention d'un support recouvert de ladite première composition
   ledit solvant étant compatible avec ledit soluté et ladite surface hydrophobe,
b) une étape subséquente de rinçage avec une solution aqueuse dudit support hydrophobe recouvert de ladite première composition pour l'obtention d'un support hydrophobe recouvert de ladite première composition rincée, et
c) une étape de mise en contact dudit support hydrophobe recouvert de ladite première composition rincée avec de l'acide hyaluronique libre pour l'obtention d'une surface présentant de l'acide hyaluronique libre,
d) une étape de réticulation de l'acide hyaluronique libre présent sur la surface obtenue à l'étape précédente à l'aide d'un agent de réticulation pour obtenir une première couche d'acide hyaluronique réticulé, et
e) une étape de mise en contact de la première couche d'acide hyaluronique réticulé avec une solution d'acide hyaluronique libre ;
où ledit agent de réticulation est un chélate de titane soluble et stable dans l'eau, notamment un chélate de formule 1 suivante : 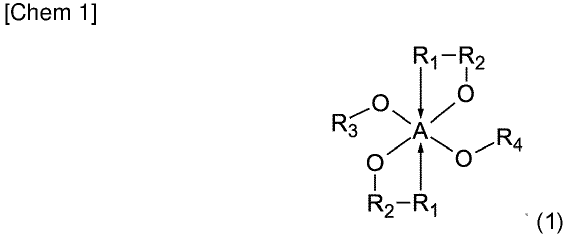 où A est Ti et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique.

Encore plus avantageusement, l'invention concerne une méthode de lubrification d'un support hydrophobe comprenant :
a) une étape de recouvrement du support avec une première composition comprenant un solvant, et
   un soluté constitué essentiellement de stéarylamine, pour l'obtention d'un support recouvert de ladite première composition
   ledit solvant étant compatible avec ledit soluté et ladite surface hydrophobe,
b) une étape subséquente de rinçage avec une solution aqueuse dudit support hydrophobe recouvert de ladite première composition pour l'obtention d'un support hydrophobe recouvert de ladite première composition rincée, et
c) une étape de mise en contact dudit support hydrophobe recouvert de ladite première composition rincée avec de l'acide hyaluronique libre pour l'obtention d'une surface présentant de l'acide hyaluronique libre,
d) une étape de réticulation de l'acide hyaluronique libre présent sur la surface obtenue à l'étape précédente à l'aide d'un agent de réticulation pour obtenir une première couche d'acide hyaluronique réticulé, et
e) une étape de mise en contact de la première couche d'acide hyaluronique réticulé avec une solution d'acide hyaluronique libre ;
où ledit agent de réticulation est un chélate de titane ou de zirconium soluble et stable dans l'eau, notamment un chélate de formule 1 suivante : 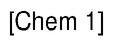 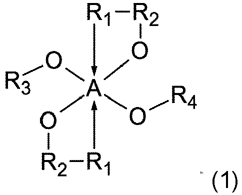 où A est Ti et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique.

Il peut être avantageux que l'étape d) susmentionnée soit suivie d'une étape de lavage avec une solution aqueuse. Cette étape de lavage permet d'éliminer l'agent réticulant en excès et hydrolyse les parties d'agent réticulant qui n'ont pas encore réagi avec l'acide hyaluronique.

Dans un autre mode de réalisation, l'étape de réticulation d) est réalisée à une température variant de 40 à 70°C, à pression atmosphérique, notamment de 45 à 65°C, en particulier à 60°C. Cette étape de chauffage augmente la réactivité de l'agent de réticulation.

Hors du contexte de l'invention, il est décrit une méthode de lubrification d'un support hydrophobe comprenant :
a) une étape de recouvrement du support avec une première composition comprenant un solvant, et
   un soluté constitué essentiellement d'acide stéarique, pour l'obtention d'un support recouvert de ladite première composition
   ledit solvant étant compatible avec ledit soluté et ladite surface hydrophobe,
b) une étape subséquente de rinçage avec une solution aqueuse dudit support hydrophobe recouvert de ladite première composition pour l'obtention d'un support hydrophobe recouvert de ladite première composition rincée, et
c) une étape de mise en contact dudit support hydrophobe recouvert de ladite première composition rincée avec du chitosan libre pour l'obtention d'une surface présentant du chitosan libre, ledit chitosan libre étant en solution à pH 5.5,
d) une étape de mise en contact de ladite surface présentant du chitosan libre avec une solution d'acide hyaluronique libre pour obtenir une surface recouverte d'acide hyaluronique,
e) une étape de réticulation de l'acide hyaluronique libre présent sur la surface obtenue à l'étape précédente à l'aide d'un agent de réticulation pour obtenir une première couche d'acide hyaluronique réticulé, et
f) une étape de mise en contact de la première couche d'acide hyaluronique réticulé avec une solution d'acide hyaluronique libre ;
où ledit agent de réticulation est un chélate de titane ou de zirconium soluble et stable dans l'eau, notamment un chélate de formule 1 suivante : 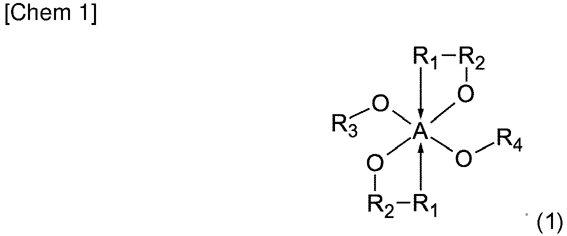 où A est Ti et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique.

Dans le contexte susmentionné, la méthode où la couche de d'acide stéarique est recouverte par du chitosan libre, il peut être avantageux après l'étape c) de réticuler ledit chitosan libre avec un agent de réticulation de formule 1 susmentionnée.

Les surfaces hydrophobes à recouvrir et à lubrifier par le procédé de l'invention sont généralement en matériaux polymères du type thermoplastique comme le polyéthylène (PE), le polypropylène (PP), le polyéthylènetéréphtalate (PET), le polybutylène théréphtalate (PBT), le polyoxyméthylène (POM), le polyarylate (PAr), polyéthercétone (PEK), les polymères fluorés (par exemple : polyfluorure de vinylidène (PVDF), les polymères cyclo-oléfiniques (COC) (par exemple les polymères commercialisés sous la marque « TOPAS »), les copolymères cyclooléfiniques (COP) (par exemple les copolymères commercialisés sous la marque «Zeonex»), polytétrafluoroéthylène (PTFE), le polychlorotrifluoroéthylène (PCTFE)), polysulfone (PSU), l'éthylène-propylène-diène monomère (EPDM), ou de type thermodurcissable comme les caoutchoucs synthétiques (par exemple: les caoutchouc halogénobutyles, les caoutchoucs nitriles, les polyisoprènes, et les polychloroprène) ou encore du type élastomères thermoplastiques (par exemple : le copolymère EPDM-PP commercialisé sous la marque « Santoprène », le copolymère bloc styrène-éthylène-butylène-styrène (SEBS), etc.).

D'autres surfaces telles que l'acier inoxydable, l'or, le titane, le platine, l'aluminium, l'alliage de nickel et de titane ou nitinol, le tantale, les silicones peuvent être recouvertes et lubrifiée selon le procédé selon l'invention. La liste susmentionnée n'est pas limitative, et l'homme du métier comprend qu'il s'agit des surfaces hydrophobes utilisables notamment dans le cadre des dispositifs à usage médical.

L'invention concerne également la méthode de lubrification d'un support hydrophobe susmentionnée, où ledit agent de réticulation est l'agent de réticulation de formule 1a1 suivante : 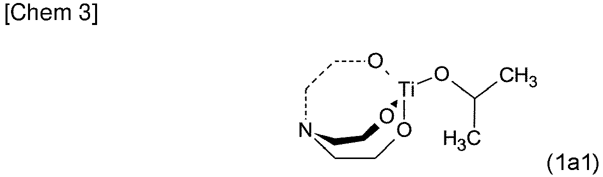 Ou de formule 1a2 suivante :

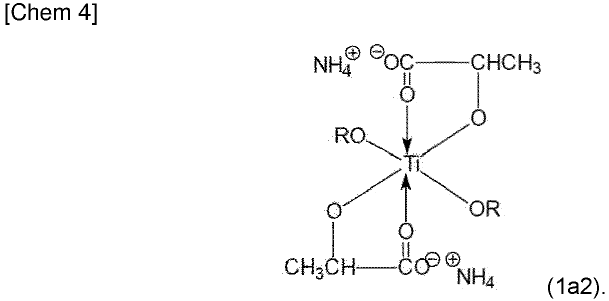

Avantageusement, l'invention concerne la méthode de lubrification d'une surface hydrophobe susmentionnée, où les étapes d) et e) sont répétées au moins une fois.

De manière avantageuse, lorsque le chitosan est utilisé, ce sont les étapes e) et f) qui sont répétées au moins 1 fois.

Dans un autre aspect, l'invention concerne un support hydrophobe lubrifié susceptible d'être obtenu selon la méthode définie ci-dessus.

Avantageusement, l'invention concerne un support hydrophobe recouvert d'une première couche de stéarylamine ou d'acide stéarique, ladite première couche de stéarylamine ou d'acide stéarique étant recouverte d'une première couche d'acide hyaluronique réticulé par est un chélate de titane-soluble et stable dans l'eau, notamment un chélate de formule 1 suivante : 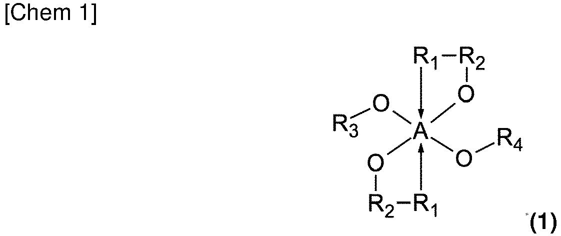 où A est Ti et
où R1 est un groupe fonctionnel contenant un atome d'oxygène ou d'azote, R2 représente deux ou trois atomes de carbone, et R3 et R4 représentent un alkyl en C1-C4 linéaire ou branché ou cyclique,
ladite première couche d'acide hyaluronique réticulé étant recouverte d'une deuxième couche d'acide hyaluronique libre.

Dans un autre mode de réalisation, l'invention concerne un support hydrophobe recouvert d'une première couche de stéarylamine, ladite première couche de stéarylamine étant recouverte d'une première couche d'acide hyaluronique réticulé par est un chélate de titane, notamment un chélate de formule 1 suivante : 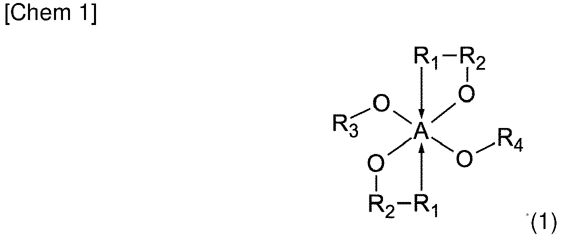 où A est Ti ou Zr ou Hf, notamment de formule 1a suivante : 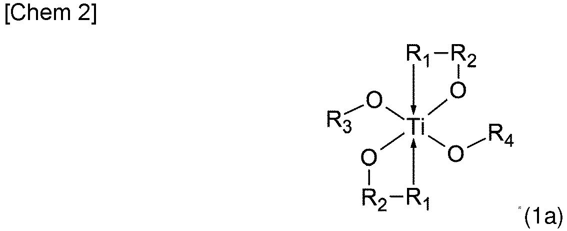 et
où R1 est un groupe fonctionnel contenant un atome d'oxygène ou d'azote, R2 représente deux ou trois atomes de carbone, et R3 et R4 représentent un alkyl en C1-C4 linéaire ou branché ou cyclique,
ladite première couche d'acide hyaluronique réticulé étant recouverte d'une deuxième couche d'acide hyaluronique libre.

En dehors du cadre de l'invention, on peut décrire ici un support hydrophobe recouvert d'une première couche d'acide stéarique, ladite première couche de d'acide stéarique étant recouverte d'une première couche de chitosan, ladite première couche de chitosan étant recouverte d'une première couche d'acide hyaluronique réticulé par est un chélate de titane ou de zirconium soluble et stable dans l'eau, notamment un chélate de formule 1 suivante : 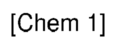 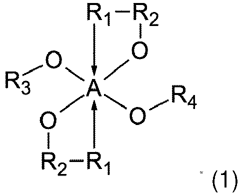 où A est Ti, notamment de formule 1a suivante 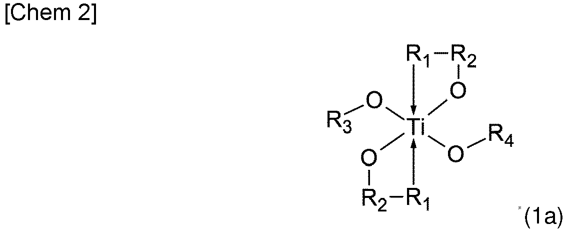 où R1 est un groupe fonctionnel contenant un atome d'oxygène ou d'azote, R2 représente deux ou trois atomes de carbone, et R3 et R4 représentent un alkyl en C1-C4 linéaire ou branché ou cyclique,
ladite première couche d'acide hyaluronique réticulé étant recouverte d'une deuxième couche d'acide hyaluronique libre.

Avantageusement, ledit agent de réticulation est le composé de formule 1 a1 susmentionné.

L'invention concerne également un kit comprenant :
a. une première composition essentiellement constituée de stéarylamine ou d'acide stéarique,
b. une seconde composition comprenant ou essentiellement constituée d'acide hyaluronique ou de chitosan, notamment de concentration de 0,1 à 0,5% m/v dans l'eau, et
c. un composé réticulant qui est un chélate de titane ou de zirconium soluble et stable dans l'eau, notamment un chélate de formule 1 suivante : 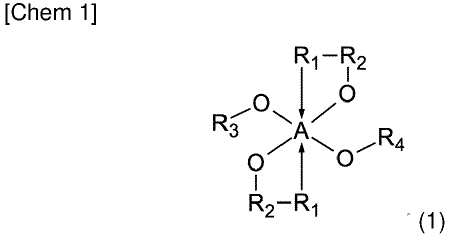 où A est Ti, notamment de formule 1a suivante : 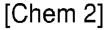 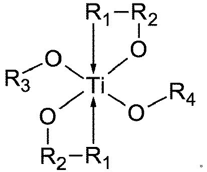 et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique, notamment le composé de formule 1 a1 suivante : 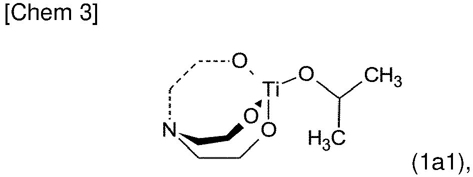 ou de formule 1a2 suivante 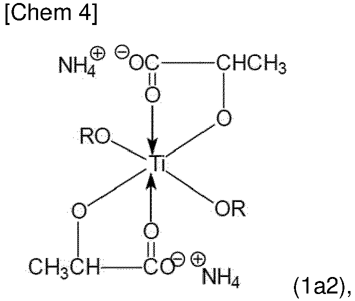 ou encore un composé de formule 1b suivante

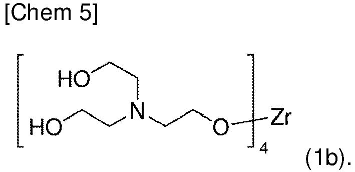

L'invention sera mieux comprise à la lumière des exemples et des figures suivantes.

### Brève description des figures

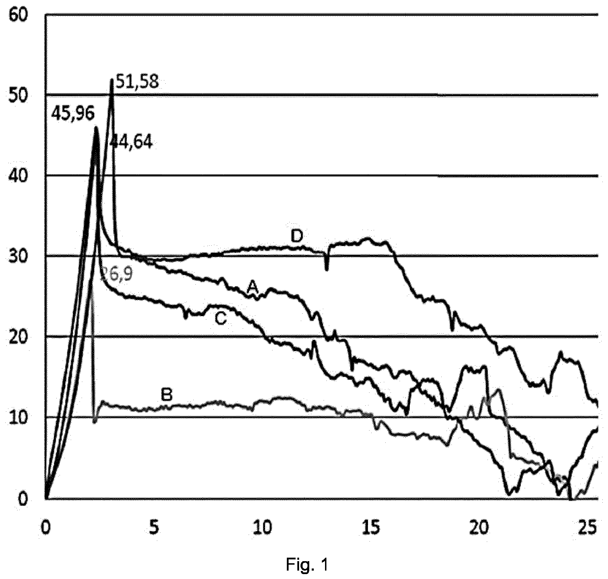

**[****Fig. 1****]** représente un graphique montrant la force (en Newtons - N) à appliquer pour déplacer le *stent* dans la capsule en fonction du déplacement (en mm). A : représente une capsule recouverte de stéarylamine et d'acide hyaluronique non réticulé ; B : représente une capsule recouverte de stéarylamine et d'acide hyaluronique réticulé avec du Tyzor TE 200mM ; C : représente une capsule recouverte de stéarylamine et d'acide hyaluronique réticulé avec du 1,4 - Butanediol Diglycidyl Ether (BDDE) 200mM, et D : représente une capsule recouverte de stéarylamine et d'acide hyaluronique réticulé avec du Poly(éthylène glycol) Diglycidyl Ether (PEGDGE) 200mM.

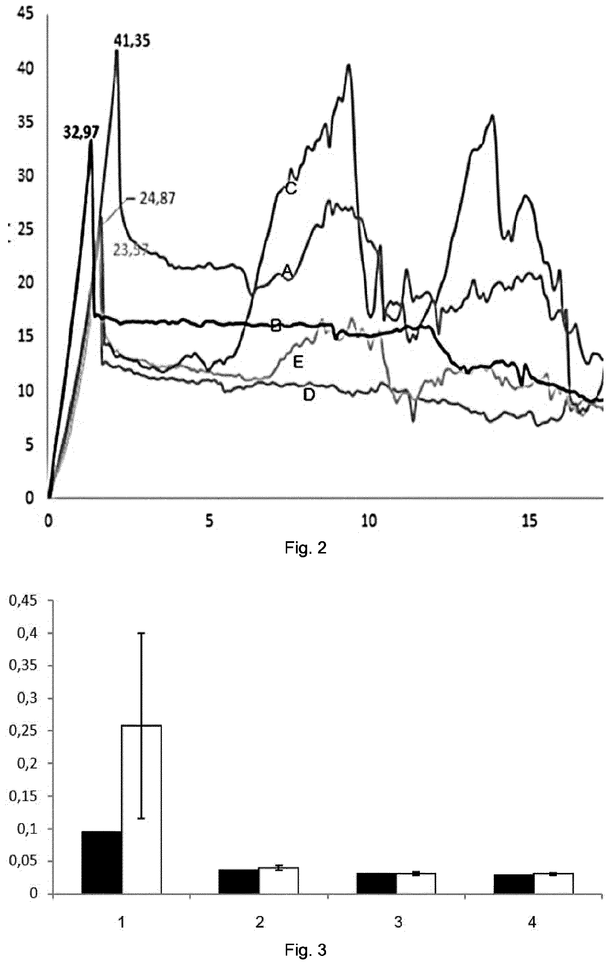

**[****Fig. 2****]** représente un graphique montrant la force (en Newtons - N) à appliquer pour déplacer le *stent* dans la capsule en fonction du déplacement (en mm). A : représente les résultats obtenus pour une capsule recouverte de stéarylamine et d'acide hyaluronique réticulé avec 50mM de Tyzor TE ; B : représente les résultats obtenus pour une capsule recouverte de stéarylamine et d'acide hyaluronique réticulé avec 100mM de Tyzor TE ; C : représente les résultats obtenus pour une capsule recouverte de stéarylamine et d'acide hyaluronique réticulé avec 200mM de Tyzor TE ; D : représente les résultats obtenus pour une capsule recouverte de stéarylamine et d'acide hyaluronique réticulé avec 400mM de Tyzor TE et D : représente les résultats obtenus pour une capsule recouverte de stéarylamine et d'acide hyaluronique réticulé avec 800mM de Tyzor TE.

**[****Fig. 3****]** représente un graphique montrant les forces (en N) pour la glissance (barres noires) et la résistance (barres blanches) sur des sondes en polyéthylène recouvertes d'acide hyaluronique selon le procédé de l'invention et soumises au passage de mâchoires de 50g sur 8cm de sonde. 1 : représente les résultats obtenus pour une sonde recouverte de stéarylamine et d'acide hyaluronique non réticulé; 2 : représente les résultats obtenus pour une sonde recouverte de stéarylamine et d'acide hyaluronique réticulé avec 50mM de Tyzor TE ; 3 : représente les résultats obtenus pour une sonde recouverte de stéarylamine et d'acide hyaluronique réticulé avec 200mM de Tyzor TE 4 : représente les résultats obtenus pour une capsule recouverte de stéarylamine et d'acide hyaluronique réticulé avec 800mM de Tyzor TE. Les résultats représentent la moyenne et l'écart-type de plusieurs essais.

### Exemples

### Exemple 1

### Recouvrement de surface hydrophobe en métal

Quatre capsules en métal (nitinol) sont lavées au préalable par immersion verticalement dans un bécher contenant de l'éthanol à 95%. Le bécher comprenant les capsules ont subi un passage aux ultrasons pendant 2 min puis le bécher est laissé dans un bain-marie à 60°C pendant 1 heure. Les capsules sont retirées de la solution de lavage et laissées à sécher à l'air libre. Ensuite, les capsules sont trempées verticalement dans un bécher contenant 80 mL de 0.1% (m/v) de stéarylamine dans le diméthylformamide. Le bécher comprenant les capsules est placé dans une étuve thermostatée à 27°C sur un agitateur orbital pendant 1 heure. Au bout de ce temps, les capsules sont retirées du bécher et elles sont trempées dans 3 bains successifs d'eau distillée pour le lavage. Enfin, les capsules sont trempées verticalement dans un bécher contenant 80 mL d'acide hyaluronique 0,2% (m/v) dans l'eau distillée. Le bécher est agité sur un agitateur orbital pendant 2 heures à température ambiante. Finalement, les capsules sont retirées et laissées à sécher à l'air libre pendant 24 heures.

### Réticulation

Quatre capsules sont recouvertes l'acide hyaluronique suivant le procédé ci-dessus.

La couche d'acide hyaluronique sera réticulée par divers agents de réticulation :
Capsule 1 (E1) : non-réticulé,
Capsule 2 (E2) : par Triéthanolamine titanate chélate - TYZOR TE 200mM,
Capsule 3 (E3) : par 1,4 - Butanediol Diglycidyl Ether (BDDE) 200mM, et
Capsule 4 (E4) : par Poly(éthylène glycol) Diglycidyl Ether (PEGDGE) 200mM.

Les trois capsules en métal E2, E3, E4, sont trempées verticalement et respectivement dans les tubes contenant 8 mL de TYZOR TE 200 mM dans l'eau distillée; 8mL de BDDE 200mM dans NaOH 0.25N et 8mL de PEGDGE 200mM dans NaOH 0.25N, ces solutions recouvrent complètement les capsules en métal à traiter.

Les tubes sont placés sur un agitateur orbital pendant 1 heure à température ambiante. Au bout de ce temps, les capsules sont retirées de la solution de l'agent réticulant et placées dans une étuve à 60°C pendant 60 min pour E2 et 15 min pour E3 et E4. Ensuite, les tubes de métal sont lavés à l'eau distillée sur un agitateur orbital pendant 15 min à température ambiante. Quatre bains de lavage successifs ont suivi. Enfin, les capsules sont trempées verticalement dans un bécher contenant 80 mL d'acide hyaluronique 0,2% (m/v) dans l'eau distillée. Le bécher est agité sur un agitateur orbital pendant 2 heures à température ambiante.

Le procédé de réticulation peut être renouvelé une deuxième fois.

Les capsules sont trempées dans un bécher contenant 80mL d'acide hyaluronique 0,3% (m/v) dans l'eau distillée.

Pour évaluer l'effet des agents réticulants, les inventeurs ont mesuré la force de dévêtissage. Pour cela, les inventeurs ont poussé à l'intérieur de la capsule traitée un *stent* tout en mesurant la force nécessaire pour le faire progresser. C'est la force de dévêtissage.

Les résultats obtenus pour les différentes réticulations sont présentés en **Figure 1****.**

Le tableau 1 suivant récapitule les résultats de la Figure 1.

**[Tableau 1]**

| **Capsule** | **Force de dévêtissage (N)** |
|---|---|
| **E1** - HA non réticulé | 45.96 |
| **E2** - HA réticulé par TYZOR TE 200mM | 26.90 |
| **E3** - HA réticulé par BDDE 200mM | 44.64 |
| **E4** - HA réticulé par PEGDGE 200mM | 51.58 |

D'après les données obtenues, la capsule donnant de meilleurs résultats est la capsule E2 où seuls 26,9N sont nécessaires pour déplacer un *stent.*

Les capsules E1 et E3 ont donné des résultats légèrement inférieurs à une capsule non traitée (pas de recouvrement ; non montré) : une moyenne de 45,3N pour ces deux capsules contre 53,7N pour une non-traitée.

La capsule E4 donne des résultats équivalents à une capsule non traitée (51,6N contre 53,7N)

Les agents réticulants ne sont donc pas équivalents, et le TYZOR TE est le meilleur agent de réticulation permettant une bonne lubrification de la surface.

### Exemple 2

Dans cet exemple, des capsules sont recouvertes comme indiqué à l'exemple 1, dans la section « Recouvrement de surface hydrophobe ».

### Réticulation

Cinq capsules sont recouvertes l'acide hyaluronique suivant le procédé ci-dessus.

La couche d'acide hyaluronique est réticulée par le TYZOR TE avec différentes concentrations :
Capsule 1 (E1a) : par TYZOR TE 50mM,
Capsule 2 (E2a) : par TYZOR TE 100mM,
Capsule 3 (E3a) : par TYZOR TE 200mM,
Capsule 4 (E4a) : par TYZOR TE 400mM, et
Capsule 5 (E5a) : par TYZOR TE 800mM.

Les cinq capsules en métal E1a, E2a, E3a, E4a et E5a sont trempées verticalement et respectivement dans les tubes contenant 8mL de TYZOR TE 50, 100, 200, 400 et 800mM dans l'eau distillée, ces solutions recouvrent complètement les capsules en métal à traiter. Les tubes sont placés sur un agitateur orbital pendant 1 heure à température ambiante. Au bout de ce temps, les capsules sont retirées de la solution de l'agent réticulant et placés dans une étuve à 60°C pendant 60 min. Ensuite, les tubes de métal sont lavés à l'eau distillée sur un agitateur orbital pendant 15 min à température ambiante. Quatre bains de lavage successifs ont été effectués. Enfin, les capsules sont trempées verticalement dans un bécher contenant 80 mL d'acide hyaluronique 0,2% (m/v) dans l'eau distillée. Le bécher est agité sur un agitateur orbital pendant 2 heures à température ambiante.

Le procédé de réticulation peut être renouvelé une deuxième fois. Dans ce cas, les capsules sont trempées dans un bêcher contenant 80mL de l'acide hyaluronique 0,3% (m/v) dans l'eau distillée.

### Résultats

Pour évaluer l'effet des agents réticulant, les inventeurs ont mesuré la force de dévêtissage. Pour cela, les inventeurs ont poussé à l'intérieur de la capsule traitée un *stent* tout en mesurant la force nécessaire pour le faire progresser. C'est la force de dévêtissage.

Les résultats obtenus pour les différentes réticulations sont présentés en **Figure 2****.**

Le tableau 2 suivant récapitule les résultats de la [Fig. 2].

**[Tableau 2]**

| **Capsule** | **Force de dévêtissage (N)** |
|---|---|
| **E1a** - HA réticulé par TYZOR TE 50mM | 41.3 |
| **E2a** - HA réticulé par TYZOR TE 100mM | 33.0 |
| **E3a** - HA réticulé par TYZOR TE 200mM | 26.0 |
| **E4a** - HA réticulé par TYZOR TE 400mM | 24.5 |
| **E5a** - HA réticulé par TYZOR TE 800mM | 23.6 |

L'efficacité de la glissance est dose-dépendante de la concentration de l'agent réticulant (TYZOR TE). Plus l'agent de réticulation est présent, plus la glissance permet de maintenir la couche d'acide hyaluronique non réticulé.

### Exemple 3

Recouvrement de surface hydrophobe en polymère

Des sondes en polyéthylène sont lavées au préalable par immersion verticalement dans un bécher contenant de l'éthanol à 95%. Le bécher comprenant les sondes a été passé aux ultrasons pendant 2 min puis le bécher est laissé dans un bain-marie à 60°C pendant 1 heure. Les sondes sont retirées de la solution de lavage et laissées à sécher à l'air libre. Ensuite, les sondes sont immergées verticalement dans un bécher contenant une solution de 0.1% (m/v) de stéarylamine dans le diméthylformamide. Le bécher comprenant les sondes est placé dans une étuve thermostatée à 27°C sur un agitateur orbital pendant 1 heure. Au bout de ce temps, les sondes sont retirées du bécher et elles sont trempées dans 3 bains successifs d'eau distillée pour le lavage. Enfin, les sondes sont immergées verticalement dans un bécher contenant une solution d'acide hyaluronique 0,2% (m/v) dans l'eau distillée. Le bécher est agité sur un agitateur orbital pendant 2 heures à température ambiante. Finalement, les sondes sont retirées et laissées à sécher à l'air libre pendant 24 heures.

Les sondes sont recouvertes l'acide hyaluronique suivant le procédé décrit ci-dessus. Les sondes sont divisées en 4 lots.

La couche d'acide hyaluronique sera réticulée par le TYZOR TE avec différentes concentrations :
Lot 1 : non-réticulé,
Lot 2 : par TYZOR TE 50mM,
Lot 3 : par TYZOR TE 200mM, et
Lot 4 : par TYZOR TE 800mM,

Les sondes de lot 2, 3 et 4 sont immergées verticalement et respectivement dans les tubes contenant une solution de TYZOR TE 50mM, 200mM et 800mM dans l'eau distillée; ces solutions recouvrent complètement les sondes en polyéthylène à traiter.

Les tubes sont placés sur un agitateur orbital pendant 1 heure à température ambiante. Au bout de ce temps, les sondes sont retirées de la solution de l'agent réticulant et placées dans une étuve à 60°C pendant 60 min. Ensuite, les sondes sont lavées à l'eau distillée sur un agitateur orbital pendant 15 min à température ambiante. Quatre bains de lavage successifs ont suivi. Enfin, les sondes sont immergées verticalement dans un bécher contenant une solution d'acide hyaluronique 0,2% (m/v) dans l'eau distillée. Le bécher est agité sur un agitateur orbital pendant 2 heures à température ambiante.

Le procédé de réticulation est renouvelé une deuxième fois.

Enfin, les sondes de lot1 (non-réticulé) ; lot 2, 3 et 4 (réticulé) sont immergées dans un bécher contenant une solution d'acide hyaluronique 0,3% (m/v) dans l'eau distillée.

Pour évaluer l'effet des agents réticulants, les inventeurs ont réalisé des tests de glissance et/ou résistance avec un passage de mâchoires de 50g sur 8cm de sondes hydratées par immersion dans l'eau distillée juste avant la mesure.

Les coefficients de friction de Glissance (CoF Glissance) sont estimés par une moyenne des coefficients du 1er passage de mâchoires.

Les coefficients de friction de Résistance (CoF Résistance) correspondent à la moyenne de 5 passages sur la même sonde.

Les résultats sont montrés à la Figure 3, et dans le tableau suivant :

**[Tableau 3]**

| | ***CoF Glissance*** | ***Ecart type*** | ***CoF Résistance*** | ***Ecart type*** |
|---|---|---|---|---|
| Lot 1 | 0,0946 | // | 0,2577 | 0,1418 |
| Lot 2 | 0,0363 | // | 0,0401 | 0,0037 |
| Lot 3 | 0,0307 | // | 0,0309 | 0,0021 |
| Lot 4 | 0,0281 | // | 0,0304 | 0,0017 |

Les résultats montrent que la réticulation de la couche d'acide hyaluronique améliore nettement la glissance et très nettement la résistance du revêtement même après plusieurs passages de friction, comparativement à la sonde témoin.

## Revendications

1. Méthode de lubrification d'un élément essentiellement constitué d'acide hyaluronique réticulé, ladite méthode comprenant
a) une étape de mise en contact dudit élément essentiellement constituée d'acide hyaluronique réticulée avec une solution d'acide hyaluronique libre,
ledit élément essentiellement constitué d'acide hyaluronique réticulé ayant été réticulé au moyen d'un agent de réticulation qui est un chélate de titane ou de zirconium soluble et stable dans l'eau, notamment un chélate de formule 1 suivante : 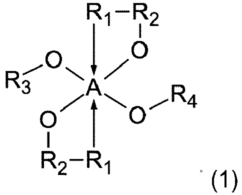
Où A est Ti, et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique.

2. Méthode selon la revendication 1, où ledit agent de réticulation est choisi parmi les agents de réticulation suivants :

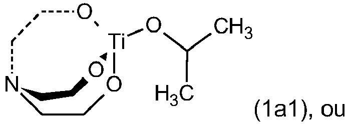

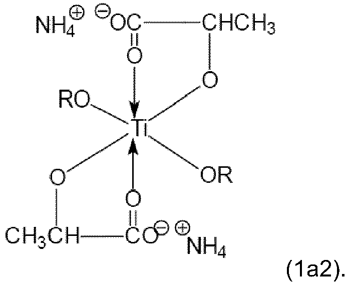

3. Méthode de lubrification selon la revendication 1 ou 2, où ledit agent de réticulation est l'agent de réticulation de formule 1a1 suivante :

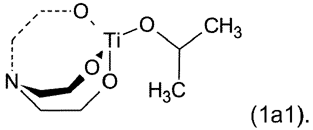

4. Méthode de lubrification selon l'une quelconque des revendications 1 à 3, comprenant, en outre :
b) une étape de réticulation de l'acide hyaluronique libre mis en contact avec ladite composition essentiellement constituée d'acide hyaluronique réticulée à l'aide d'un agent de réticulation de formule 1 pour obtenir une seconde couche de composition essentiellement constituée d'acide hyaluronique réticulé, et
c) une étape de mise en contact de la seconde couche de composition essentiellement constituée d'acide hyaluronique réticulé avec une solution d'acide hyaluronique libre.

5. Méthode de lubrification selon la revendication 4, où les étapes b) et c) sont répétées au moins une fois.

6. Élément lubrifié essentiellement constituée d'au moins une couche d'acide hyaluronique réticulé par un agent de réticulation qui est un chélate de titane soluble et stable dans l'eau, ladite au moins une couche d'acide hyaluronique réticulé étant recouverte d'une couche d'acide hyaluronique non réticulé, ledit élément lubrifié étant susceptible d'être obtenu selon le procédé défini à l'une quelconque des revendications 1 à 5.

7. Méthode de lubrification d'un support hydrophobe comprenant :
a. une étape de recouvrement du support avec une première composition comprenant
- un solvant, et
- un soluté choisi parmi la stéarylamine ou l'acide stéarique, pour l'obtention d'un support recouvert de ladite première composition ledit solvant étant compatible avec ledit soluté et ladite surface hydrophobe,
b. une étape subséquente de rinçage avec une solution aqueuse dudit support hydrophobe recouvert de ladite première composition pour l'obtention d'un support hydrophobe recouvert de ladite première composition rincée, et
c. une étape de mise en contact dudit support hydrophobe recouvert de ladite première composition rincée avec de l'acide hyaluronique libre pour l'obtention d'une surface présentant de l'acide hyaluronique libre,
d. une étape de réticulation de l'acide hyaluronique libre présent sur la surface obtenue à l'étape précédente à l'aide d'un agent de réticulation pour obtenir une première couche d'acide hyaluronique réticulé, et
e. une étape de mise en contact de la première couche d'acide hyaluronique réticulé avec une solution d'acide hyaluronique libre ;
où ledit agent de réticulation est un chélate de titane ou de zirconium soluble et stable dans l'eau, notamment un chélate de formule 1 suivante : 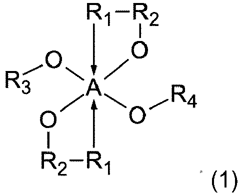
Où A est Ti, et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique.

8. Méthode de lubrification d'une surface hydrophobe selon la revendication 7 les étapes d) et e) sont répétées au moins une fois.

9. Support hydrophobe lubrifié susceptible d'être obtenu selon la méthode selon l'une quelconque des revendications 7 ou 8, notamment recouvert d'une première couche de stéarylamine ou d'acide stéarique, ladite première couche de stéarylamine ou d'acide stéarique étant recouverte d'une première couche d'acide hyaluronique réticulé par est un chélate de titane ou de zirconium soluble et stable dans l'eau, notamment un chélate de formule 1 suivante : 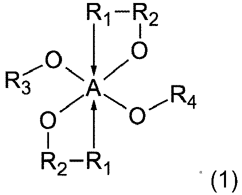 où A est Ti, et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique,
ladite première couche d'acide hyaluronique réticulé étant recouverte d'une deuxième couche d'acide hyaluronique libre.

10. Kit comprenant :
a. une première composition essentiellement constituée de stéarylamine ou d'acide stéarique,
b. une seconde composition comprenant ou essentiellement constituée d'acide hyaluronique, et
c. un composé réticulant, ledit composé réticulant étant un chélate de titane ou de zirconium soluble et stable dans l'eau, notamment un chélate de formule 1 suivante 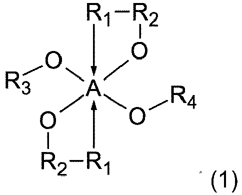
où A est Ti, et
où R₁ est un groupe fonctionnel contenant un atome d'oxygène ou d'azote, R₂ représente deux ou trois atomes de carbone, et R₃ et R₄ représentent un alkyl en C₁-C₄ linéaire ou branché ou cyclique, notamment le composé de formule 1a1 : 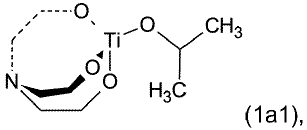 ou de formule 1a2 suivante :

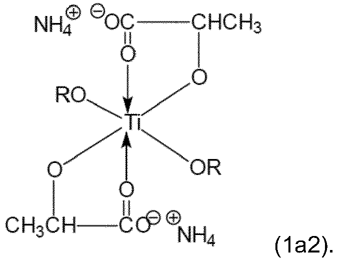

## Patentansprüche

1. Verfahren zum Schmieren eines Elements, das im Wesentlichen aus vernetzter Hyaluronsäure besteht, das Verfahren umfassend
a) einen Schritt eines Inberührungbringens des Elements, das im Wesentlichen aus vernetzter Hyaluronsäure besteht, mit einer Lösung aus freier Hyaluronsäure,
wobei das Element, das im Wesentlichen aus vernetzter Hyaluronsäure besteht, mittels eines Vernetzungsmittels vernetzt wurde, das ein lösliches und in Wasser stabiles Titan- oder Zirkoniumchelat ist, insbesondere ein Chelat der folgenden Formel 1: 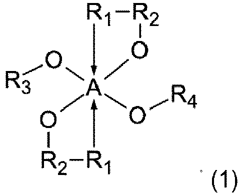
wobei A Ti ist, und
wobei R₁ eine funktionelle Gruppe ist, die ein Sauerstoff- oder Stickstoffatom enthält, R₂ zwei oder drei Kohlenstoffatome darstellt und R₃ und R₄ ein lineares oder verzweigtes oder zyklisches C₁-C₄-Alkyl darstellen.

2. Verfahren nach Anspruch 1, wobei das Vernetzungsmittel aus den folgenden Vernetzungsmitteln ausgewählt ist: 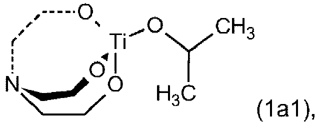 oder

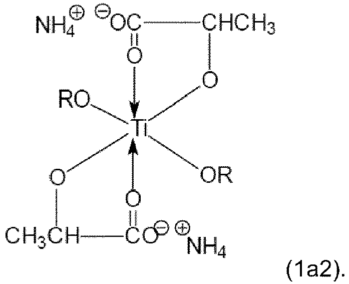

3. Schmierverfahren nach Anspruch 1 oder 2, wobei das Vernetzungsmittel das Vernetzungsmittel der folgenden Formel 1a1 ist:

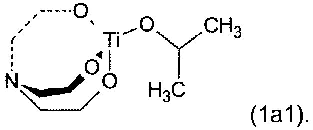

4. Schmierverfahren nach einem der Ansprüche 1 bis 3, umfassend ferner:
b) einen Schritt des Vernetzens der freien Hyaluronsäure, die mit der Zusammensetzung in Berührung gebracht wird, die im Wesentlichen aus vernetzter Hyaluronsäure besteht, mittels eines Vernetzungsmittels der Formel 1 zum Erhalten einer zweiten Schicht der Zusammensetzung, die im Wesentlichen aus vernetzter Hyaluronsäure besteht, und
c) einen Schritt des Inberührungbringens der zweiten Schicht der Zusammensetzung, die im Wesentlich aus vernetzter Hyaluronsäure besteht, mit einer Lösung aus freier Hyaluronsäure.

5. Schmierverfahren nach Anspruch 4, wobei die Schritte b) und c) mindestens einmal wiederholt werden.

6. Geschmiertes Element, das im Wesentlichen aus mindestens einer Schicht aus Hyaluronsäure besteht, die durch ein Vernetzungsmittel vernetzt ist, das ein lösliches und in Wasser stabiles Titanchelat ist, wobei die mindestens eine Schicht aus vernetzter Hyaluronsäure mit einer Schicht aus nicht vernetzter Hyaluronsäure bedeckt ist, wobei das geschmierte Element geeignet ist, um gemäß dem Verfahren erhalten zu werden, das in einem der Ansprüche 1 bis 5 definiert ist.

7. Verfahren zum Schmieren eines hydrophoben Trägers, umfassend:
a. einen Schritt des Bedeckens des Trägers mit einer ersten Zusammensetzung, umfassend
- ein Lösungsmittel, und
- einen gelösten Stoff, der aus Stearylamin oder Stearinsäure ausgewählt ist, zum Erhalten eines Trägers, der mit der ersten Zusammensetzung bedeckt ist
wobei das Lösungsmittel mit dem gelösten Stoff und der hydrophoben Oberfläche kompatibel ist,
b. einen anschließenden Schritt eines Spülens mit einer wässrigen Lösung des hydrophoben Trägers, der mit der ersten Zusammensetzung bedeckt ist, zum Erhalten eines hydrophoben Trägers, der mit der gespülten ersten Zusammensetzung bedeckt ist, und
c. einen Schritt des Inberührungbringens des hydrophoben Trägers, der mit der gespülten ersten Zusammensetzung bedeckt ist, mit der freien Hyaluronsäure zum Erhalten einer Oberfläche, die freie Hyaluronsäure aufweist,
d. einen Schritt des Vernetzens der freien Hyaluronsäure, die auf der Oberfläche vorliegt, die in dem vorhergehenden Schritt erhalten wird, mittels eines Vernetzungsmittels zum Erhalten einer ersten Schicht aus vernetzter Hyaluronsäure, und
e. einen Schritt des Inberührungbringens der ersten Schicht aus vernetzter Hyaluronsäure mit einer Lösung aus freier Hyaluronsäure;
oder wobei das Vernetzungsmittels ein lösliches und in Wasser stabiles Titan- oder Zirkoniumchelat ist, insbesondere ein Chelat der folgenden Formel 1: 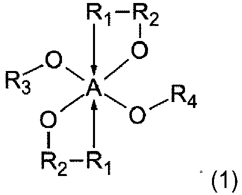
wobei A Ti ist, und
wobei R₁ eine funktionelle Gruppe ist, die ein Sauerstoff- oder Stickstoffatom enthält, R₂ zwei oder drei Kohlenstoffatome darstellt und R₃ und R₄ ein lineares oder verzweigtes oder zyklisches C₁-C₄-Alkyl darstellen.

8. Verfahren zum Schmieren einer hydrophoben Oberfläche nach Anspruch 7, wobei die Schritte d) und e) mindestens einmal wiederholt werden.

9. Geschmierter hydrophober Träger, der geeignet ist, um gemäß dem Verfahren nach einem der Ansprüche 7 oder 8 erhalten zu werden, der insbesondere mit einer ersten Schicht aus Stearylamin oder Stearinsäure bedeckt ist, wobei die erste Schicht aus Stearylamin oder Stearinsäure, die mit einer ersten Schicht aus Hyaluronsäure bedeckt ist, die vernetzt ist durch, ein lösliches und in Wasser stabiles Titan- oder Zirkoniumchelat ist, insbesondere ein Chelat der folgenden Formel 1: 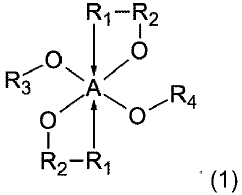 wobei A Ti ist, und
wobei R₁ eine funktionelle Gruppe ist, die ein Sauerstoff- oder Stickstoffatom enthält, R₂ zwei oder drei Kohlenstoffatome darstellt und R₃ und R₄ ein lineares oder verzweigtes oder zyklisches C₁-C₄-Alkyl darstellen,
wobei die erste Schicht aus vernetzter Hyaluronsäure mit einer zweiten Schicht aus freier Hyaluronsäure bedeckt ist.

10. Kit, umfassend:
a. eine erste Zusammensetzung, die im Wesentlichen aus Stearylamin oder Stearinsäure besteht,
b. eine zweite Zusammensetzung, umfassend oder im Wesentlichen bestehend aus Hyaluronsäure, und
c. eine vernetzende Verbindung, wobei die vernetzende Verbindung ein lösliches und in Wasser stabiles Titan- oder Zirkoniumchelat ist, insbesondere ein Chelat der folgenden Formel 1: 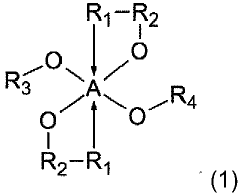
wobei A Ti ist, und
wobei R₁ eine funktionelle Gruppe ist, die ein Sauerstoff- oder Stickstoffatom enthält, R₂ zwei oder drei Kohlenstoffatome darstellt und R₃ und R₄ ein lineares oder verzweigtes oder zyklisches C₁-C₄-Alkyl darstellen, insbesondere die Verbindung der Formel 1a1: 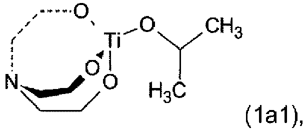
oder der folgenden Formel 1a2:

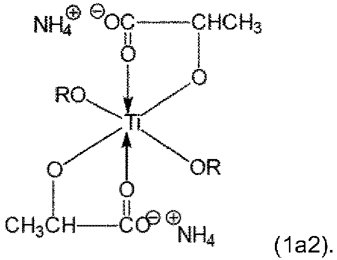

## Claims

1. A method for lubricating an element essentially consisting of crosslinked hyaluronic acid, said method comprising
a) bringing said element essentially consisting of crosslinked hyaluronic acid into contact with a solution of free hyaluronic acid,
said element essentially consisting of crosslinked hyaluronic acid being previously crosslinked by means of a crosslinking agent which is a chelate of titan or zirconium which is soluble and stable in water, preferably a chelate of following formula 1: 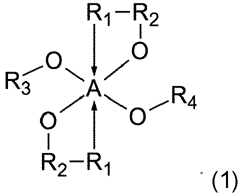
wherein A is Ti, and
wherein R₁ is a functional group comprising an Oxygen or Nitrogen atom, R₂ represents two or three atoms of Carbon, and R₃ and R₄ represent a C₁-C₄ linear or branched or cyclic alkyl.

2. The method according to claim 1, wherein said crosslinking agent is chosen from the following crosslinking agents: 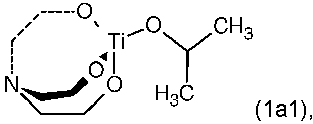 or

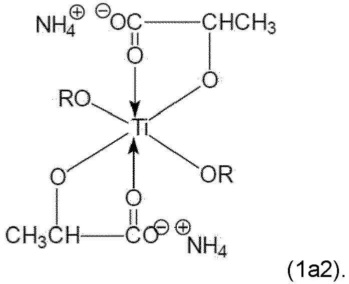

3. The method for lubrificating according to claim 1 or 2, wherein said crosslinking agent is the crosslinking agent of following formula 1a1:

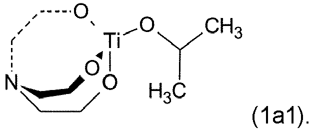

4. The method for lubricating according to anyone of claims 1 à 3, further comprising:
b) crosslinking the free hyaluronic of the lubricated element by using a crosslinking agent of formula 1 in order to obtain a second layer of composition essentially consisting of crosslinked hyaluronic acid, and
c) bringing the second layer of composition essentially consisting of crosslinked hyaluronic acid into contact with a solution of free hyaluronic acid, in order to obtain an element covered by a second layer of composition which is lubricated.

5. The method of lubricating according to claim 4, wherein steps b) and c) are repeated at least once.

6. A lubricated element essentially consisting of at least one layer of hyaluronic acid crosslinked by a crosslinking agent which is a titanium chelate that is soluble and stable in water, said at least one layer of crosslinked hyaluronic acid being covered by a layer of non-crosslinked hyaluronic acid, said lubricated element being liable to be obtained by the method as defined in anyone of claims 1 to 5.

7. A method for lubricating a hydrophobic support comprising:
a. covering the hydrophobic support with a first composition comprising
- a solvent, and
- a solute selected from stearylamine or stearic acid, to obtain a support covered with
said first composition, said solvent being compatible with said solute and said hydrophobic surface,
b. rinsing said hydrophobic support covered with said first composition with an aqueous solution in order to obtain a hydrophobic support covered with said first rinsed composition, and
c. bringing said hydrophobic support covered with said first rinsed composition into contact with free hyaluronic acid in order to obtain a surface having free hyaluronic acid,
d. crosslinking the free hyaluronic acid present on the surface obtained in the previous step by using a crosslinking agent in order to obtain a first layer of crosslinked hyaluronic acid, and
e. bringing the first layer of crosslinked hyaluronic acid into contact with a solution of free hyaluronic acid in order to obtain a lubricated hydrophobic support;
wherein said crosslinking agent is titanium chelate or zirconium chelate soluble and stable in water, preferably a chelate off following formula 1: 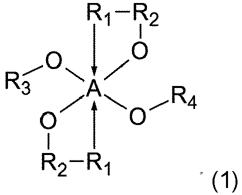
wherein A is Ti, and
wherein R₁ is a functional group comprising an Oxygen or Nitrogen atom, R₂ represents two or three atoms of Carbon, and R₃ and R₄ represent a C₁-C₄ linear or branched or cyclic alkyl.

8. The method for lubricating a hydrophobic support according to claim 7 wherein steps d) and e) are repeated at least once.

9. A lubricated hydrophobic liable to be obtained by the method as defeined in claim 7 or 8, preferably covered with a first layer of stearylamine or stearic acid, said first layer of stearylamine or stearic acid being covered by a first layer of hyaluronic acid crosslinked by a crosslinking agent which is a titanium chelate or zirconium chelate soluble and stable in water, in particular a chelate of following formula 1: 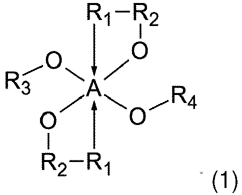 wherein A is Ti, and
wherein R₁ is a functional group comprising an Oxygen or Nitrogen atom, R₂ represents two or three atoms of Carbon, and R₃ and R₄ represent a C₁-C₄ linear or branched or cyclic alkyl,
said first layer of hyaluronic acid crosslinked by a crosslinking agent being covered by said second layer of free hyaluronic acid.

10. A kit comprising:
a. a first composition essentially consisting of stearylamine or stearic acid,
b. a second composition comprising or essentially consisting of hyaluronic acid, and
c. a crosslinking compound, said crosslinking compound being a titanium chelate or zirconium chelate soluble and stable in water, in particular a chelate of following formula 1: 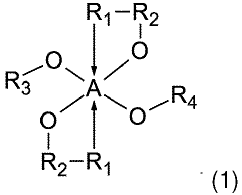
wherein A is Ti, and
wherein R₁ is a functional group comprising an Oxygen or Nitrogen atom, R₂ represents two or three atoms of Carbon, and R₃ and R₄ represent a C₁-C₄ linear or branched or cyclic alkyl, in particular a compound of following formula 1a1: 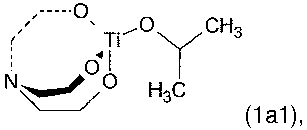
or of following formula 1 a2:
